# EUROPEAN PATENT SPECIFICATION

(11) **EP 1 335 742 B1**
(45) Date of publication and mention of the grant of the patent: **23.07.2008**
(21) Application number: 01916008.4
(22) Date of filing: 15.03.2001
(51) Int. Cl.: A61K 38/02, A61K 39/00

(54) **Use of peptides dervied from apoB100 in the production of a vaccine/diagnostic against atherosclerosis**
Verwendung von apoB100 Peptidderivaten zur Herstellung eines Impfstoffs/Diagnose gegen Atherosklerose
Utilisation de peptides dérivés d'apoB100 dans la production d'un vaccin/diagnostic contre l'athérosclérose

(30) Priority: 15.03.2000 SE 0000855
(43) Date of publication of application: 20.08.2003
(73) Proprietor: Forskarpatent i Syd AB, 223 70 Lund (SE)
(72) Inventor: HANSSON, Göran, K., S-113 28 Stockholm (SE); STEMME, Sten, S-181 30 Lidingö (SE); NICOLETTI, Antonino, F-75015 Paris (FR); WUTTGE, Dirk, S-172 30 Sundbyberg (SE)
(74) Representative: Larfeldt, Helene
(86) International application number: PCT/SE2001/000570
(87) International publication number: WO 2001/068119

(56) References cited:
- WULF PALINSKI ET AL.: 'Immunization of low density lipoprotein (LDL) receptor-deficient rabbits with homologous malondialdehyde-modified LDL reduces atherogenesis' PROC. NATL. ACAD. SCI. USA vol. 92, January 1995, pages 821 - 825, XP002941307
- JACOB GEORGE ET AL.: 'Hyperimmunization of apo-E-deficient mice with homologous malondialdehyde low-density lipoprotein suppresses early atherogenesis' ARTERIOSCLEROSIS vol. 138, 1998, pages 147 - 152, XP002941306
- WULF PALINSKI ET AL.: 'Antisera and monoclonal antibodies specific for epitopes generated during oxidative modification of low density lipoprotein' ARTERIOSCLEROSIS vol. 10, no. 3, May 1990 - June 1990, pages 325 - 335, XP002941308
- GORAN K. HANSSON: 'Cell-mediated immunity in atherosclerosis' CURRENT OPINION IN LIPIDOLOGY vol. 8, 1997, pages 301 - 311, XP002941309

## Description

### Technical field

The present invention relates to an antigenic composition useful for immunization of mammals against atherosclerosis. Further, the invention also relates to methods of producing such an antigenic composition.

### Background

Atherosclerosis is a degenerative disease affecting the entire human population. At present, atherosclerosis is treated either by lowering the circulating cholesterol level (and consequently the LDL level), e.g. by specific lipid-lowering drugs and diets, or alternatively by counteracting the modifications which are considered to make the LDL more atherogenic, e.g. by antioxidants by eliminating other risc factors for atherosclerosis, such as hypertension and smoking, or by surgical removal of atherosclerotic tissue. However, in spite of these treatments, atherosclerosis is still rated on the WHO list of the most fatal diseases in the world, thus indicationg a strong need for a more efficient therapy or, more preferred, even a method of preventing actual outbreak of the disease by vaccination.

A substantial research effort has of the above mentioned reasons been directed towards finding a cure for atherosclerosis. More specifically, the involvement of cellular immunity in atherosclerosis has been established (Stemme S, Holm J, Hansson GK. T lymphocytes in human atherosclerotic plaques are memory cells expressing CD45RO and the integrin VLA-1. Arterioscler. Thromb. 1992;12:206-211; Stemme S, Rymo L, Hansson GK. Polyclonal origin of T lymphocytes in human atherosclerotic plaques. Lab. Invest. 1991;65:651-660), but its target is still a matter of debate. Further, it has recently been shown that T cells extracted from the human plaque recognize oxidized low density lipoprotein (oxLDL) (Stemme S, Faber B, Holm J, Wiklund O, Witzum JL, Hansson GK. T lymphocytes from human atherosclerotic plaques recognize oxidized low density lipoprotein. Proc. Natl. Acad. Sci. USA 1995;92:3893-3897). However, since the reacting T cells are largely HLA class II restricted TcRαβ⁺ cells (Stemme S, Faber B, Holm J, Wildund O, Witzum JL, Hansson GK. T lymphocytes from human atherosclerotic plaques recognize oxidized low density lipoprotein. Proc. Natl. Acad. Sci. USA 1995;92:3893-3897), it is likely that the T cell antigens are derived from proteins rather than from lipids. The nature of the antigens has however not been established. As there is no reactivity against the native protein of the LDL particles, apoB100, according to one theory, T cells could recognize a self protein which has been modified in a way that changes its processing, resulting in a different pattern of cleavage into peptides. Alternatively, in another theory, the modified protein could carry new T cell epitopes not present during negative thymic selection, and thus be recognized by non-deleted T cell clones. The immunogenic potential of proteins modified by the oxidatively derived aldehydes MDA and HNE is demonstrated by a recent study where MDA- and HNE-modified albumin induced T cell responses in mice (Wuttge D, Bruzelius M, Stemme S: T cell recognition of lipid peroxidation products breaks tolerance to self proteins. Immunology 1999; 98:273-279).

Palinski et al (Proc. Natl. Acad. Sci., Volume 92, January 1995: "Immunization of low density lipoprotein (LDL) receptor-deficient rabbits with homologous malondialdehyde-modified LDL reduces atherogenesis", p. 821-825) have shown that immunization with MDA-LDL can reduce atherosclerosis in hypercholesterolemic rabbits. Even though high-titer antibodies to MDA-LDL were developed, no analysis of T cell responses to MDA-LDL was performed. The T cell content in atherosclerotic lesions was analysed, but no differences were observed between immunized and control rabbits. Palinski et al speculate that immunization may have led to both humoral and cell-mediated immune responses, but no data are provided concerning characteristics of any potential T cell epitopes or antigen recognition mechanism. Accordingly, Palinski et al do not provide the information required for creating an antigenic composition useful for vaccine purpose.

Further, George et al (Atherosclerosis, Volume 138, 1998: "Hyperimmunization of apo-E-deficient mice with homologous malondialdehyde low-density lipoprotein suppresses early atherogenesis", p. 147-152) have shown that immunization with MDA-LDL reduces atherosclerosis in apo-E-deficient mice. Similar to the findings of the above discussed Palinski article, it was observed that high-titer antibodies were formed against MDA-LDL. No effect was observed on the T cell composition in the atherosclerotic lesions. However, this study fails to observe any differences in the content of CD4 and CD8 lymphocytes in the lesions of both study groups and concludes that it is less likely the possibility of a local immune modulation of the plaque size by MDA-LDL-specific T lymphocytes.

In summary, even though it has been known that oxidized LDL will cause an immunogenic response in a mammal, since LDL is a highly heterogenic protein, it will give rise to a plurality of various responses if administered *in vivo,* thus rendering it inapplicable for use *per se* in a vaccine composition. Accordingly, in order to provide an efficient vaccine against atherosclerosis, firstly, it needs to be established which is the target for the subject cellular immunity, and secondly, an immunogenic composition may be formulated based on such findings regarding the structure of the T cell epitopes.

### Summary of the present invention

The present invention provides an explanation to the mammalian immunogenic response to an oxidized LDL by defining antigenic compositions capable of binding the T cell receptor (TcR) active therein. Thus, the present invention relates to such an antigenic composition for use as a medicament as well as to the use thereof in the manufacture of a pharmaceutical composition, such as a vaccine.

### Brief description of the drawings

Figure 1 shows the proliferation of LN cells from apoB-TG mice immunized with human oxLDL in response to native and oxidized human LDL.
Figure 2 shows that ApoB-TG hybridomas recognize oxLDL in an I-A^{b} restricted manner. Data are proliferation of (the IL-2 and IL-4 sensitive) CTL-L2 cell line when incubated with culture supernatants from T cell hybridomas exposed to native or oxidized LDL in the presence of irradiated, syngeneic (I-A^{b}) antigen-presenting spleen cells.
Figure 3 A-E show how hybridoma 96-7 expresses TcRαβ with Vβ6 variable domains.
Figure 4 shows the nucleotide and extrapolated amino acid sequences of the CDR3 region (grey box) of the 96-7 hybridoma.
Figure 5 shows the 3D structure of the TcR of the clone 96-7 discussed in more detail below.
Figure 6 shows that hybridoma 96-7 recognizes MDA-apoB100.
Figure 7 shows that T cell hybridomas derived from non-immunized apoE KO mice recognize modified LDL.
Figure 8A shows that hybridoma M20 recognizes MDA-bound peptides both when presented after uptake of MDA-albumin and when conjugated to surface proteins of fixed antigen-presenting cells.
Figure 8B shows that antigen recognition by hybridoma M20 could be inhibited with an antibody to MDA-Lysine.

### Definitions

Unless defined otherwise, all technical and scientific terms used herein have the same meanings as commonly understood by one of ordinary skill in the art to which this invention belongs. For the purpose of clarity, some terms used in the present application will be defined below.
In the present application, the term "low density lipoprotein" (LDL) refers to the the lower density form of lipoprotein present in serum. Such lipoproteins are classified according to their density into chylomicrons, very low density lipoproteins (VLDL), LDL and high density lipoproteins (HDL). They are particles comprising proteins (apolipoproteins) associated with lipids and have a micelle-like spherical structure composed of a non-polar core consisting of triacylglycerol and cholesteryl ester on one hand and apolipoproteins, cholesterol and phospholipid covering the core on the other hand. Apolipoproteins and lipids are different according to each lipoprotein. More specifically, LDL has a particle size of about 180 to 280 Ångstroms in diameter and a density within a range of 1.006 to 1.063 (g/ml), while its apolipoprotein is composed mainly of apoB-100. LDL is a normal blood constituent that is the body's principal means for delivery of cholesterol to tissues.
The term" oxLDL" refers to an oxidized form of LDL.
An "antigen" means a substance, such as a foreign substance, that, when introduced into the body, can stimulate an immune response. Thus, an antigenic composition is any composition comprising such an antigen, i.e. together with a suitable carrier.
The term complementary determining region (CDR) means the regions of a T cell receptor (TcR) that are genetically hypervariable. By virtue of their unique amino acid sequences, they project unique chemical structures at the surface of the TcR and thereby determine the chemical properties of the antigenic structure that will be recognized by each T cell.
As used herein, the term "vaccine" means any compound or preparation of antigens designed to stimulate a primary immune response, resulting in proliferation of the memory cells and the ability to exhibit a secondary memory or anamnestic response upon subsequent exposure to the same antigens.
In the present application, the term "adjuvant" means any substance or compound capable of promoting an immune response in a mammalian species, e.g. by modifing the activities of cells that are concerned with generating and maintaining the immune response or by modifying the presentation of antigen to the immune system.
The term "monoclonal antibody" as used herein, refers to a population of antibody molecules that contain only one species of an antigen binding site capable of immunoreacting with a particular epitope of an antigen. A monoclonal antibody composition thus typically displays a single binding affinity for a particular protein with which it immunoreacts.
"Library" means a pool containing a large number of different molecules, such as peptides or molecules of peptidic structure, typically comprised of different sequences or subunits, or a combination of different sequences of side chains and linkages, or differently substituted molecules.
The term "comprises" means herein including but not limited to.

### Detailed description of the invention

More specifically, the present application relates to an antigenic composition comprised of a peptide-aldehyde conjugate, wherein the peptide is derived from the protein apoB100, or is of LDL-origin. The composition is capable of eliciting an immune response against non-native, oxidized low density protein (LDL) in a subject by interacting with T cell receptors (TcR). This is the first time that a role for T cell receptors are identified for the present purpose, which is highly surprising, especially in view of the above discussed findings by Palinski et al, and by George et al, where in fact the published data argue against an effect of immunisation on T cell activity.

The aldehyde portion of the present conjugate is in the most preferred embodiment an oxidatively produced aldehyde. Such an oxidation may e.g. be performed by exposure to oxidizing agents, like copper, iron etc., and will result in peroxidation of the lipid components, and covalent modifications of the protein components of LDL.

More specifically, the aldehyde portion is a dialdehyde, such as malondialdehyde (MDA). However, other dialdehydes may be contemplated, such as oxaldialde-hyde, succindialdehyde, glutardialdehyde, adipic dialdehyde, pimelic dialdehyde etc. In an alternative embodiment, the aldehyde portion of the present conjugate is a modified monoaldehyde, such as 4-hydroxynonenal (4-HNE).

Further, the peptide portion of the present conjugate preferably includes at least one lysine residue. In a specific embodiment, the peptide portion contains several proline residues that promote the formation of an MHC-binding secondary structure. Accordingly, the present peptide portion differs from the LDL particles used e.g. by Palinski, as discussed above, since the present invention only utilises the protein component of LDL particles, in the form of a well defined peptide. As mentioned above, LDL particles are comprised of protein, phospholipids, cholesterol, cholesteryl esters and triglycerides. (For a review of the heterogeneous molecular family of LDLs, see e.g. Krauss R. M. and Burke D. J. J. Lipid. Res. 23 97-104 (1982)). As the skilled person in this field will realise, such a heterogenous composition as an LDL particle cannot be envisaged for practical use e.g. in a vaccine composition for human use, since the number of various responses that can be elicited thereby is not possible to foresee. Thus, one great advantage with the present invention is that it provides an antigenic composition which contrary to the prior art is capable of eliciting a well defined response.

In the most preferred embodiment, the antigenic properties are enhanced by association with a major histocompatibility (MHC) antigen. As is well known in this field, the MHC is a series of genes that encode protein molecules needed for immune responses. The MHC of mammalian species contains three group of genes: class I, class II and class III, wherein class I and II MHC products control recognition of self and non-self. Even though MHC molecules are specifically discussed in the present application, it is to be understood that the same general principles will also apply to HLA molecules, thus also included within the scope of the invention. As discussed in detail in the Experimental section below, the present invention demonstrates for the first time that the immune response against an oxidized LDL in a mammal is restricted to MHC Class II, as T cells of the I-A^{b} haplotype recognized oxidized LDL presented by I-A^{b} expressing but not I-A^{d} expressing antigen-presenting cells. Thus, the present immunisation is a self recognizing reaction. Thus, by combining the peptide-aldehyde conjugate with the appropriate MHC II region, the most efficient composition according to the invention is obtained. This is particularly true as in general, T lymphocytes only recognize the antigen and respond to it when presented on the surface of the antigen-presenting cell.

Accordingly, the present invention shows for the first time a dependence on MHC molecules for immunisation and protection tothis end.

In the most preferred embodiment, said TcR chains are α10 and β6 chains essentially of the sequence disclosed in SEQ ID NO:4 and SEQ ID NO:2, respectively, or any functional derivative, fragment or analogue thereof having similar antigenic properties. In other words, a "functional" derivative, fragment or analogue means herein that such a derivative, fragment or analogue is also capable of interacting with the antigenic composition according to the invention. Testing such elements regarding their respective function is easily performed by the skilled in this field using common general knowledge and routine methods. Thus, even though the sequences relate to TcR chains originating from mouse, it is to be understood that the present invention also relates to any antigenic peptide-aldehyde composition similar to the one disclosed above, which is capable of providing an interaction leading to an immunogenic response to non-native oxidized LDLs when administered to a mammal. Thus, the variable regions of the TcRs of a human subject are expected to deviate somewhat from the present sequences, presumably by exhibiting a homology of about 40% with the mouse TcR presented herein. In general, such a homology may be at least about 60%, and preferably at least about 74%, even though it is noted that the advantage of the present invention resides resides in the herein provided evidence that the TcR that recognizes oxidized LDL is in fact of α10β6 nature. Thus, according to the present invention, it has been shown that the response of a mammal against an oxidized LDL is of peptidic rather than lipidic nature, which has hitherto never been shown before. Further, the advantageous function of the herein discosed combination of aldehyde-peptide now enables the compounds according to the invention to be prepared e.g. by the screening method disclosed below.

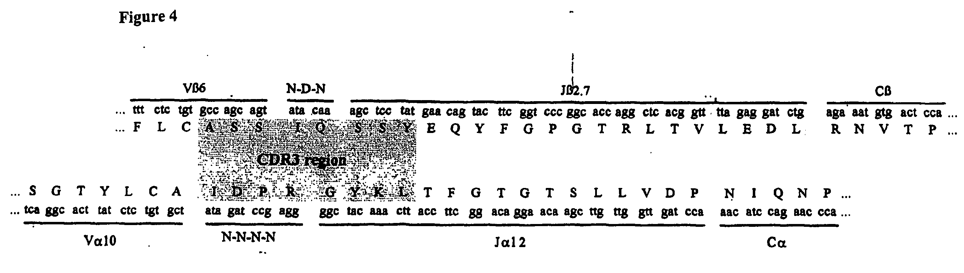

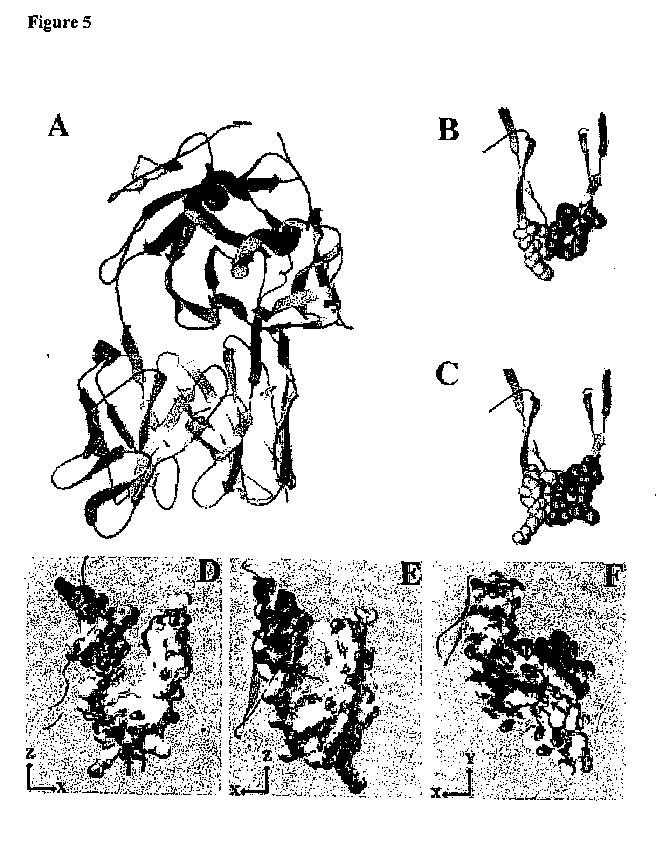

The present TcR chains are also depicted in Figure 4 wherein the mutual relationship between said two sequences is shown. Accordingly, in one embodiment, the composition according to the invention will be comprised of a spatial configuration similar or essentially identical to the three dimensional disclosure of Figure 5. More specifically, the rather flat or shallow structure of the CDR3 region together with the exposed surface charges shown appear to be essential for the antigenic properties thereof. Accordingly, one particular embodiment of the present composition exhibits the three dimensional structure of Figure 5, while the exact amino acid sequence thereof may deviate to some degree from the sequences disclosed in SEQ ID NO:4 and SEQ ID NO:2, respectively. In SEQ ID NO 3, the nucleic acid sequence encoding SEQ ID NO:4 is shown, and as regards amino acid no. 18 (Gly), it is noted that even though the third base of said triplet has been shown as a G, it may equally well be a U, C or A, which triplet will also result in the amino acid Gly. Thus, a further aspect of the invention is the nucleic acid sequences encoding the present TcRs and homologues thereof, essentially as shown in SEQ ID NO:1 and SEQ ID NO:3, as well as any sequence capable of specific hybridisation to said sequences.

More specifically, in the section "Experimental" below, the detailed characterization of one hybridoma, denoted 96-7, will be presented. Its TcR carried the Vα10/Vβ6 chains with a cluster of charged and polar amino acids in complementarity-determining region 3 (CDR3). In the present context, "a cluster of charged and polar amino acids" means that a plurality of amino acids are selected from the group consisted of charged amino acids, more specifically among cysteine, tyrosine, aspartic acid, glutamic acid, arginine, histidine and lysine, and/or the group that is consisted of polar amino acids, more specifically among serine, threonine, cysteine, tyrosine, aspartic acid, asparagine, glutamic acid, glutamine, arginine, histidine and lysine. This hybridoma recognized purified apoB100 protein conjugated with malondialdehyde (MDA) but not the native, unconjugated apoB100 protein, thus provifing evidence that the present antigenic composition should comprise a dialdehyde, such as MDA. It also reacted with oxLDL but not native LDL. Thus, T cells of apoB TG mice recognize MDA-conjugated protein components after immunization with oxLDL.

To study whether the above discussed process occurs spontaneously as part of the autoimmune response to oxLDL in atherosclerosis, T cell hybridomas from apolipoprotein E knockout (apoE KO) mice were established. Since these mice were not immunized prior to isolation of T cells, the reactivity of the hybridomas was expected to reflect autoimmune reactivity. Again, T cell hybridomas were identified that recognized oxLDL and MDA-LDL. Several different TcR-VB were expressed by these hybridomas, suggesting that autoimmunization during atherosclerosis involves reactions against several different T cell epitopes. Accordingly, in a specific embodiment thereof, the present antigenic composition is capable of eliciting immune reactions by interacting with more than one such β chain.

The present application also relates to a cell which is capable of producing the peptide portion of an antigenic composition according to the invention on the surface thereof. Such a cell may be a prokaryotic or eukaryotic cell, such as any one of a plurality of well known mammalian cell lines frequently used in genetic recombination and expression of peptides and proteins. For the production of such a cell, a DNA encoding the desired peptide together with suitable regulatory elements is introduced into a suitable vector, such as a plasmid, a virus, etc. Said vector is then used to transfect a host cell by use of any well known method depending on the nature of the vector and cell in order to express the peptide portion on the cell surface. Subsequently, a conjugation of a suitable aldehyde portion may be performed by methods well known to those of skill in the field. In case of a procaryotic cell, the above mentioned regulatory elements should include signals required for secretion of the product. In one embodiment, the present cell is a I-A^{b} haplotype and capable of I-A^{b} expression. Methods useful for the recombinant production of the present composition are e.g. found in Sambrook et al., Molecular Cloning, A Laboratory Manual, 2nd ed., vol. 1-3, Cold Spring Harbour Laboratory, Cold Spring Harbor, NY, 1989; and Culture of Animal Cells, A Manual of Basic Technique, 3rd ed., Wiley-Liss, New York, NY (1994).
Further, the present composition is easily produced by well known methods for peptide synthesis and organic chemistry, see e.g Stewart et al., Solid Phase Peptide Synthesis, 2nd ed., Pierce Chem Co., Rockford, Ill. (1984). Further, a screening method for obtaining the compositions according to the invention from a library will be described in detail below.

In addition, the present application also relates to an animal model which expresses the above described TcR, i.e. the αβ chains from mouse, or any homologue thereof derived from another species, such as human. In one advantageous embodiment, the invention relates to a rodent model, preferably mouse, which expresses the TcR essentially as defined in SEQ ID NOS: 1 and 4. The animal models according to the invention are advantageously used in vaccine preparation in order to optimise an immunogenic composition for use as a vaccine against atherosclerosis. Further, the present invention also encompasses cell cultures useful for the same purpose, preferably animal cells, e.g. a well known cell line wherein the gene encoding the present TcR α10β6 chains, or any homologue thereof, have been introduced. Accordingly, the invention also relates such cells and cell lines.

Thus, in a further aspect, the present invention relates to a method of producing a vaccine against atherosclerosis, which comprises the steps of
(a) providing a library of peptides, preferably derived from the protein apoB100 or of LDL origin;
(b) incubating said peptides with suitable aldehydes under biological conditions and a time sufficient to produce conjugates of peptide-aldehyde;
(c) separating non-conjugated peptides and aldehydes from the library;
(d) incubating the conjugates obtained as described above with a T cell clone, such as a hybridoma, that responds to an oxidized LDL in combination with an antigen-presenting cell that displays major histocompatibility complex (MHC) class II proteins essentially identical to, e.g. of the same or a similar haplotype as, the clone;
(e) selecting conjugates capable of specific activation of the T cell clone; and
(f) formulating a vaccine composition from said selected conjugate and a pharmaceutically acceptable carrier.

The vaccine produced by the method according to the invention may comprise any one of the above described embodiments of the antigenic composition.

More specifically, the components of the incubation step can be added to form a mixture in any order and way that provides the requisite bindings. The incubation temperature should enable optimal binding or conjugation, typically between 4 and 40°C, such as about 15-40⁰C, e.g. about 37⁰ C. The separation steps may be performed in a variety of ways well known to those of skill in the field, e.g. by immobilisation of one component on a solid substrate. The immobilisation may e.g. be accomplished by use of conventinal binding partners, such as biotin-streptavidin or the like and the solid substrate may be a microtiter plate, microbeads, a dipstick, resin particles or any other suitable material. Thus, such a separation may include one or more rinse or washings in order to remove unbound or unconjugated material as well as further components of the incubation mixture, e.g. salts, buffers, detergents etc. Detection may be effected in any convenient way, such as by coupling one of the components to a suitable label, e.g. a radioactive isotope of phosphorous directly detectable by particle counters.
Regarding the production of T cell hybridomas, as an example, the manufacture of a CD4+ T cell hybridoma will be disclosed below in the experimental section. (The hybridoma technique was originally described by Köhler and Milstein (Köhler and Milstein. 1975, Nature. 256:495-497).

The application also relates to a pharmaceutical preparation, i.e. a vaccine, for generating an immune response in a mammal to non-native, oxidized LDL, which preparation comprises an antigenic composition as defined above together with a pharmaceutically acceptable carrier. Optionally, it may also include and/or one or more suitable adjuvants. The antigenic composition may for example originally have been identified by the method disclosed above, even though once it has been identified, it can conveniently be produced by any method well known to the skilled in this field, such as chemical synthesis or recombinant DNA technique followed by chemical conjugation etc. (See e.g. Fields and Noble (1990), Int. J. Pept. Protein Res, 35: 161-214). In the preferred embodiment, the aldehyde portion of the composition is a dialdehyde, such as malondialdehyde (MDA), or any other dialdehyde as discussed above. In the most preferred embodiment, the present preparation is manufactured in a form suitable for subcutaneous injection. However, other forms may be encompassed, such as intravenous, cutaneous or subcutaneous, nasal, intramuscular or intraperitoneal etc. (Methods of drug delivery are discussed e.g. in Langer, Science 249:1527-1533 (1990)). Regardless of the route of administration, the preparations according to the invention are formulated into pharmaceutically acceptable dosage forms by conventional methods known to those of skill in this field (see e.g. Remington's Pharmaceutical Sciences, 16th edition, Osol, A., 1980). In the present preparation, the amount of active ingredient combined with the carrier for a single dosage form will vary depending on the host being treated, e.g. age, body weight etc, the selected route of administration and other factors well known to those of skill on this field. The pharmaceutically acceptable carrier may be any suitable aqueous or non-aqueous carrier, such as water, saline, ethanol, polyols, such as glycerol, proplylene glycol, polyethylene glycol, or the like, or any other suitable material. The present solutions are sterile and generally free of undesirable matter. Further, they may also comprise one or more suitable adjuvant(s) that enhances the effect of the vaccine, such as an aluminium salt. It may also be desirable to include isotonic agents, such as sugars, sodium chloride etc in the preparation. Further additives, such as wetting agents, emulsifying agents, dispersing agents etc are included as requisite.

In the present context, it is understood that the considerations discussed above in relation to the antigenic composition also will apply to the vaccine comprising said antigenic composition of the present method.

The application also relates to antibodies for the prevention and/or treatment of atherosclerosis. In a first embodiment, an antibody is raised against the TcR chains involved in the atherosclerotic immunogenic pathway, such as TcR chains of the sequence disclosed in SEQ. ID. NOS:4 and 2, or the human homologue thereof, or any functionally equivalent derivative, fragment or analogue thereof. Such antibodies are produced by administering the present TcR as a vaccine. Alternatively, the antibodies are produced in a first subject by exposure to the present TcR and subsequently used to treat a second subject by administration thereof as therapy. In a second embodiment, the antibody according to the invention is an antibody raised against oxidized LDL, and most preferably, it is an immunoglobulin G (IgG) antibody. Such an antibody have indeed been observed before, but the present inventors suggest for the first time its use for prevention and/or treatment of atherosclerosis, enabled as the effect and reactivity thereof has now been elucidated.

In therapy, the antibodies according to the invention will be administered in one or more dosages, and the amount needed will depend on during which phase of the disease the therapy is given as well as on other factors. In order to produce such novel antibodies, the antigenic composition according to the invention will be administered to a subject in order to induce the production of the above described antibodies characteristic for athersclerosis. Preferably, the novel antibodies will be monoclonal antibodies. Once designed, such novel antibodies may be produced by conventional techniques and used in therapy. In general, a monoclonal antibody to an epitope of the present antigen can be prepared by using a technique which provides for the production of antibody molecules from continuous cell lines in culture and methods of preparing antibodies are well known to the skilled in this field (see e.g. Coligan (1991) Current Protocols in Immunology, Wiley/Greene, NY; Harlow and Lane (1989) Antibodies: A Laboratory Manual, Cold Spring Harbor Press, NY; and Goding (1986) Monoclonal Antibodies: Principles and Practice (2nd ed) Academic Press, New York, NY). For therapeutic purposes, there may be an interest in using human antibodies. Immunisation of a human host with the T cell receptor or fragment thereof to activate T cells specific for the sequence of interest is one possibility. Alternatively, mice or other lower mammals are immunized, and the genes encoding the variable regions of the antibodies specific for the present T cell region of interest are isolated and manipulated by joining to an appropriate human constant region, and optionally, the complementary determining regions (CDR) used to replace the CDRs of a human antibody by genetic engineering. The resulting chimeric construct, comprising a lower mammal variable region or CDRs and a human constant region may then be transformed into a microorganism or mammalian host cell in culture, particularly a lymphocyte, and the hybrid antibodies expressed Also recent techniques suggest random association of immunoglobulin genes from a human host for expression in a non-human cell host e.g. prokaryotic, and screening for affinity.
For therapeutic purposes, the present antibody is formulated with conventional pharmaceutically or pharmacologically acceptable vehicles for administration, conveniently by injection. Vehicles include deionized water, saline, phosphate-buffered saline, Ringer's solution, dextrose solution, Hank's solution, etc. Other additives may include additives to provide isotonicity, buffers, preservatives, and the like. The antibody may be administered parenterally, typically intravenously or intramuscularly, as a bolus, intermittently or in a continuous regimen. Desirably, the dose should deplete or at least bind about 75% of the undesired T cells, preferably at least about 90%.

The application further relates to the use of a cell that is induced to carry MDA-protein conjugates as an immunogen for the prevention and/or treatment of atherosclerosis. In a first embodiment, cells obtained from the blood or another easily accessible source of an individual are treated with MDA to generate MDA-protein complexes on the cell surface. The protocol for MDA conjugation of cell surface proteins may or may not involve a step of fixation before the addition of MDA in order to prevent the cell from internalizing the added MDA and thus to ensure that modification is directed towards cell surface proteins alone. After modification, MDA conjugated cells will be formulated with conventional pharmaceutically or pharmacologically acceptable vehicles for administration, conveniently by injection or oral administration. Additives may include substances to preserve isotonicity, buffers, preservatives, and possibly also one or more suitable adjuvant(s) that enhances the immunogenic effect of the cell preparation. Administrations of the MDA-conjugated cells will be performed either by a single injection or by repeated injections in such a way that maximal protective immune responses reactive towards MDA-LDL are achieved in the recipient individual.

In a second embodiment, HLA class II molecules corresponding to the haplotype of an individual are generated by recombinant DNA technology involving expression in a suitable prokaryotic or eukaryotic expression system (see above) and subjected to MDA conjugation. The exposure of the HLA molecules may be optimized by incorporating them in a liposome or other compartmentalized lipid-protein particle. Such preparations of MDA-conjugated HLA molecules will be formulated for parenteral or peroral administration with or without the addition of suitable preservatives or adjuvants and used for injections. Administrations of the MDA-conjugated HLA molecule preparations will be performed either by a single injection or by repeated injections in such a way that maximal protective immune responses directed against, or reactive towards, MDA-LDL are achieved in the recipient individual.

In another aspect, the present invention relates to the use of a T cell receptor (TcR), which exhibits essential similarities with the mouse TcR identified according to the present invention as described by SEQ ID NOS:2 and 4 as well as in Figure 5, as a lead compound in a method of molecular modelling of a ligand capable of binding such a TcR. In order to successfully design an advantageous ligand, practical experimentation will preferably be combined with the modelling procedure. As compared to the above described screening method according to the invention, molecular modelling will provide ligands with equal or even enhanced therapeutic activity for use as vaccines. More specifically, novel ligands may be modelled according to physical properties, e.g. stereochemistry, bonding, size and/or charge, using data from a range of sources, e.g. spectroscopic techniques, X-ray diffraction data and NMR. Computational analysis and similarity mapping as well as other techniques are useful to this end. The TcR lead should be in the configuration adapted thereof in its active i.e. binding state. Alternatively, an antigenic composition according to the invention is used as a lead in order to prepare a peptidomimetic thereof, i.e. a molecule that closely resembles the original peptide-aldehyde conjugate but which preferably exhibits enhanced properties, such as an improved therapeutic activity, no side-effects, ease of preparation etc.

In a last aspect, the present invention relates to a method of diagnosis of atherosclerosis which comprises determining the presence in a human host of the herein defined TcR, or any functional equivalent thereof, preferably the human homologue, the presence of such TcR being an indication or marker of atherosclerosis. The present diagnosis is more advantageous than previously known methods using X-ray techniques as the present diagnosis enables a diagnosis at a much earlier stage, in turn enabling an earlier started therapy or treatment. More specifically, by analysing a simple cell or blood sample, the present invention provides a diagnostic method which is proportional rather than quantitative, as are previously known methods. The novel proportional analysis according to the invention will provide a more reliable diagnosis, as variations exist between patients due to age, body weight etc., thus making the prior art concentration based methods less exact.

Thus, the invention relates to a method for diagnosis of affliction with atherosclerosis in a patient, by which method the presence of a TcR as disclosed by SEQ ID NO:4 and SEQ ID NO:2, or a functional derivative, fragment or analogue thereof, is assayed, said method comprising the steps of:
(a) combining a sample of body fluid obtained from said patient, with a marker of the TcR to be detected;
(b) detecting the relative presence of TcR in said sample;
(c) comparing the detected presence of TcR to a reference value; and
(d) using the comparison in (d) in the diagnosis of affliction with atherosclerosis,
wherein in step (d), the proportion of the total amount of T cells which constitutes TcRs having a spatial configuration essentially as the CDR3 region disclosed in Figure 5 and comprising an amino acid sequence essentially as defined by SEQ ID NO:4 and SEQ ID NO:2, is determined.

### Detailed description of the drawings

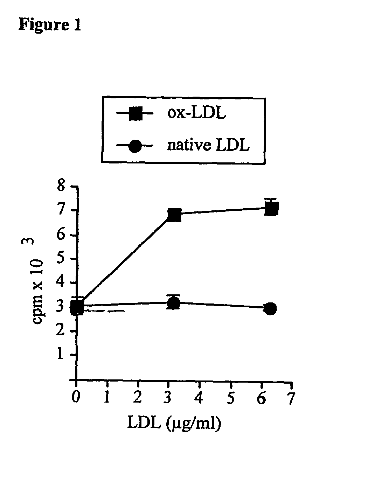

Figure 1 illustrates the proliferation of LN cells from apoB-TG mice immunized with human oxLDL in response to native and oxidized human LDL.

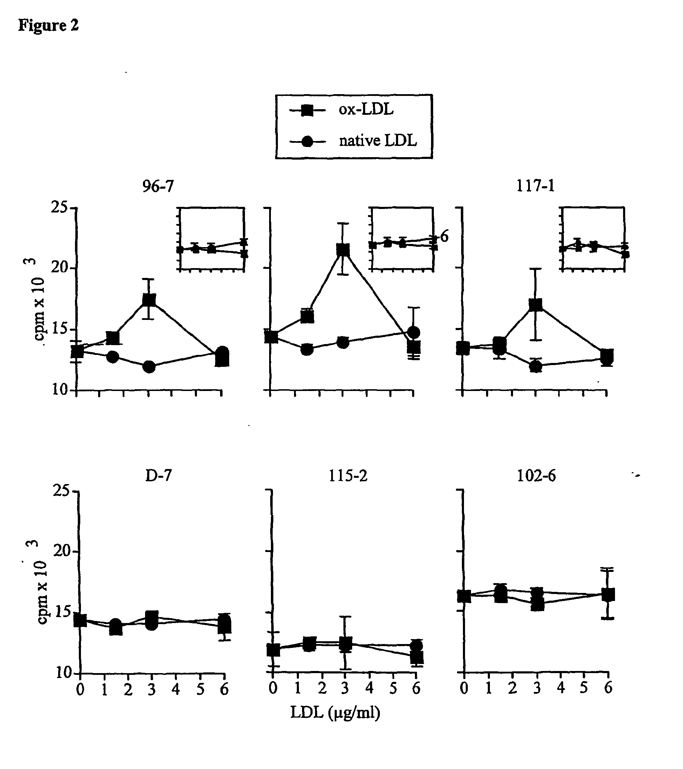

Figure 2 illustrates that ApoB-TG hybridomas recognize oxLDL in an I-A^{b} restricted manner. Data are proliferation of (the IL-2 and IL-4 sensitive) CTL-L2 cell line when incubated with culture supernatants from T cell hybridomas exposed to native or oxidized LDL in the presence of irradiated, syngeneic (I-A^{b}) antigen-presenting spleen cells. For positive hybridoma clones, activation in the presence of allogeneic I-A^{d} APCs is shown in the inset (same scale: 10.10³ - 25.10³ cpm). Approximatively 50,000 cpm and 30,000 cpm were counted with 20 U IL-2 and with supernatant from concanavalin A-stimulated (2.5 µg/ml) hybridomas, respectively.

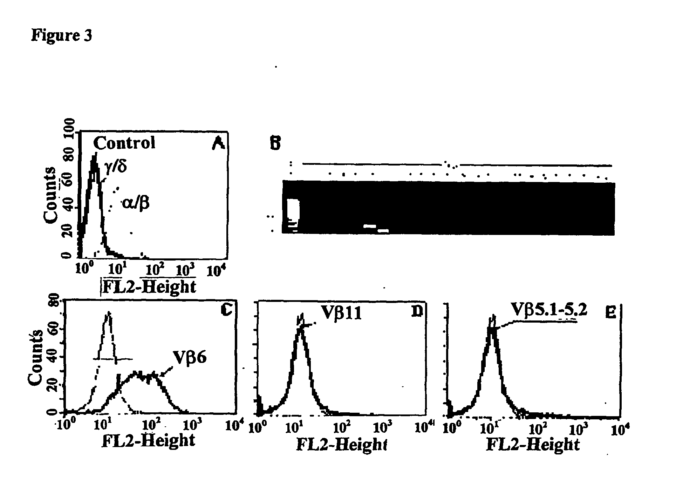

Figure 3 illustrates how hybridoma 96-7 expresses TcRαβ with Vβ6 variable domains.
   A) FACS analysis showing surface expression of TcRαβ, but not TcRγδ.
   B) RT-PCR analysis reveals expression of mRNA for variable domains Vβ6 and Vβ5.2. The latter is derived from the hybridoma fusion partner BW5147 (not shown).
   C-E) FACS analysis demonstrates surface expression of TcR- VB6 but not Vβ11 or VB5.1-5.2.
Figure 4 illustrates the nucleotide and extrapolated amino acid sequences of the CDR3 region (grey box) of the 96-7 hybridoma.
Figure 5 illustrates the 3D structure of the clone 2c (A, B; (Garcia KC, Degano M, Stanfield RL, Brunmark A, Jackson MR, Peterson PA, Teyton L, Wilson IA. An alphabeta T cell receptor structure at 2.5 A and its orientation in the TCR-MHC complex :see comments:. Science 1996;274:209-219)) and of the clone 96-7 (C-F). B-F show magnifications of the CDR3 region (position Vα 80-117; VB 84-116). D-F: The 96-7 TcR is charactarized by charged (arrows, **red color** - ändra markeringssätt så att det syns i svart-vitt) and polar amino acids and by the side chain of the arginine residue (Arg 100) protruding out of the TcR

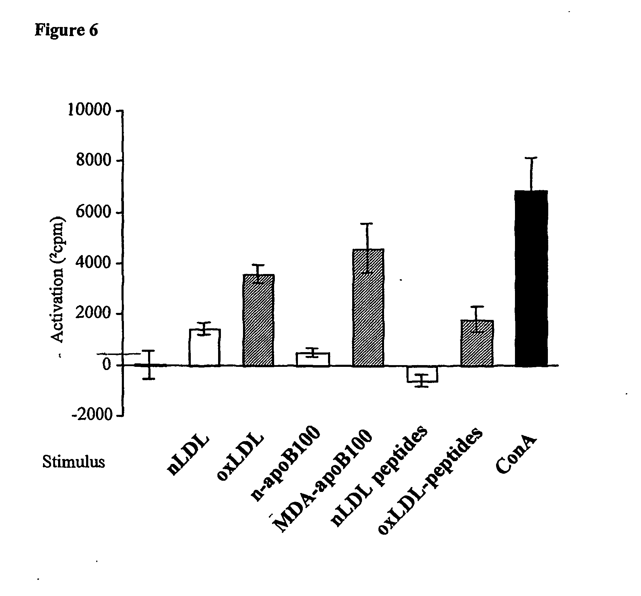

Figure 6 illustrates that hybridoma 96-7 recognizes MDA-apoB100. Activation was analyzed using irradiated syngeneic spleen cells as antigen presenting cells and the indicator cell line CTL-L2 as described in the legend to Fig. 2 and is presented as Δcpm (cpm of stimulated culture - cpm of unstimulated culture). nLDL, native LDL; oxLDL, oxidized LDL; n-apoB100, native apoB100 protein; MDA-apoB100, malondialdehyde-modified apoB100 protein; n-LDL peptides, proteolytic peptides of native LDL; oxLDL peptides, proteolytic peptides of copper-oxidized LDL. Mean ± SEM.

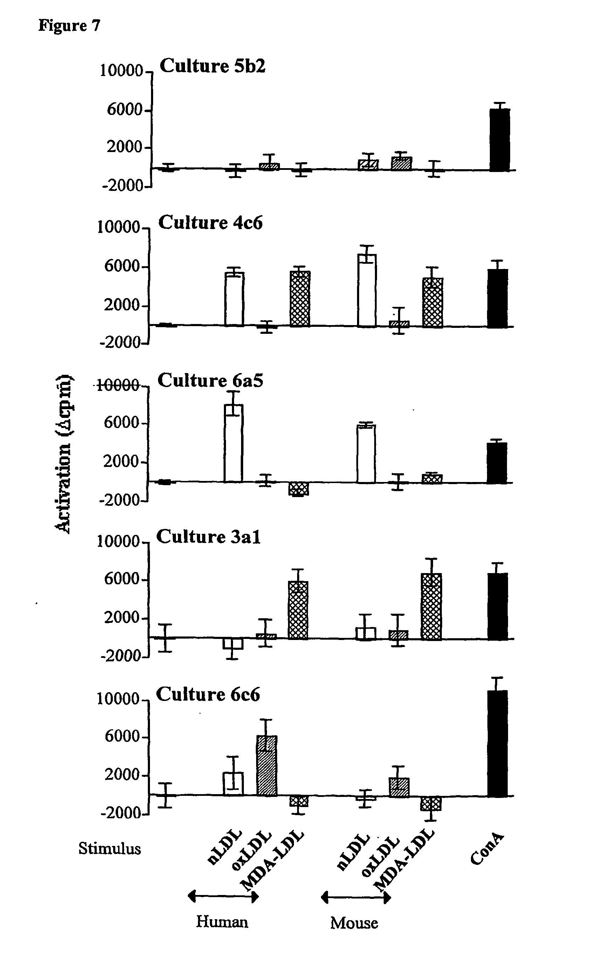

Figure 7 illustrates that T cell hybridomas derived from non-immunized apoE KO mice recognize modified LDL. The CTL-L2 assay was used to assess the reactivity of hybridomas to human and mouse oxLDL, MDA-LDL, and native LDL in the presence of syngeneic APC. Data shown are Δcpm calculated as described in the legend to Fig. 6. The following TcR-Vβ domains were identified in these cultures: Culture 6c6 - VB4, 8.2, 10, 13; culture 3a1 - Vβ1; culture 6a5 - VB2, 6, 12; culture 4c6 - VB12.

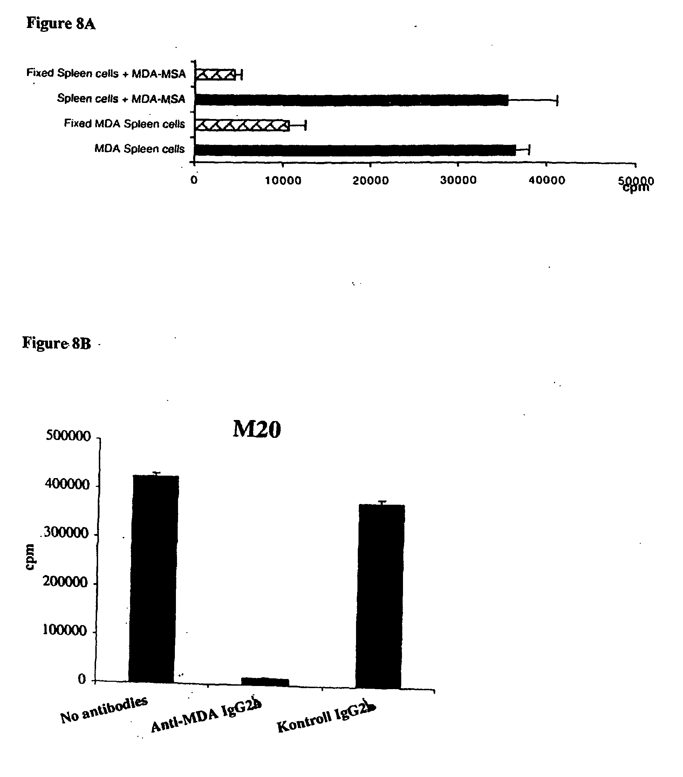

Figure 8A shows how fixed MDA-modified spleen cells stimulated hybridoma M20 (bottom bar), while unfixed, but not fixed, (spleen cells were able to process and present whole MDA-MSA (top bars). Figure 8B shows how spleen cells were pulsed with MDA-MSA, washed and coincubated with M20 hybridoma T cells. MDA-specific antibodies, but not control IgG2a antibodies, completely blocked antigen recognition by the hybridoma T cells. In summary, Fig 8 shows that (i) the MDA-adducts on peptides can be recognized without processing and (ii) the aldehyde is pointing out from the MHC-bound peptide towards the TcR. This rules out processing-dependent recognition of native self peptides and thus provides the first direct evidence that aldehyde-adducts can form part of a TcR epitope.

Further conclusions drawn by interpretation of the present drawings will be discussed below in the Experimental section.

### EXPERIMENTAL

The examples discussed below are provided only for the purpose of illustrating the present invention and should not be construed as any limitation of the scope as defined by the appended claims.

### Procedure

According to the present invention, it was hypothesized that the immunogenicity of LDL could result from the reaction of aldehydes with positive amino acids in apoB100 such as lysine. To test this possibility, two studies were performed. Firstly, the immune response of apoB100-transgenic (apoB-TG) mice to oxidized LDL was characterized. Since such mice have been engineered to produce high levels of human apoB100 apolipoprotein (Linton MF, Farese RJ, Chiesa G, Grass DS, Chin P, Hammer RE, Hobbs HH, Young SG. Transgenic mice expressing high plasma concentrations of human apolipoprotein B100 and lipoprotein(a). J. Clin. Invest. 1993;92:3029-3037), they are expected to be tolerant to native human LDL. These mice were immunized with human oxLDL and the T cell response against oxidation-induced epitopes on LDL by T cell hybridomas was characterized. Secondly, since TcR recognition of lipid peroxidation derived aldehyde adducts has previously not been described, the present inventors corroborated the findings above in a model system where normal B6 mice were immunized with MDA-albumin. Using this approach, the concept of direct TcR recognition of MDA-adducts was confirmed.

### Material and Methods

### LDL and apoB preparations

Human LDL (d=1.019-1.063 g/ml) was obtained under sterile conditions by ultracentrifugation of pooled human plasma collected from 10 donors. Human apoB100 (Sigma, St Louis, USA) was extensively dialyzed against a 0.02 µg/mL SDS solution to remove most of the SDS contained in the lyophilized preparation. The final SDS concentration was determined in separate experiments and found to be optimal at 0.02 µg/mL in order to keep the protein soluble, allow the MDA modification, and prevent cytotoxic effects. Malondialdehyde-modified LDL and apoB100 (MDA-LDL and MDA-apoB) were produced as previously described (Palinski W, Rosenfeld ME, Yl:a-Herttuala S, Gurtner GC, Socher SS, Butler SW, Parthasarathy S, Carew TE, Steinberg D, Witztum JL. Low density lipoprotein undergoes oxidative modification in vivo. Proc Natl Acad Sci USA 1989;86:1372-1376; Palinski W, Ylä-Herttuala S, Rosenfeld ME, Butler SW, Socher SA, Parthasarathy S, Curtiss LK, Witztum JL. Antisera and monoclonal antibodies specific for epitopes generated during oxidative modification of low density lipoprotein. Arterioscler. Thromb. 1990;10:325-335). Briefly, MDA was generated by acidic reaction of 1,1,3,3-tetramethoxypropane (Sigma) with HCl (4M) for 10 min at 37°C. After neutralization of the MDA solution, 100µl of MDA (0.5M) was added to 1 mg of LDL or 1 mg apoB100. After 3 hours of incubation at 37°C, free MDA was removed by dialysis. Oxidized LDL was produced by an overnight oxidation with 5 µM CuSO₄ as previously described (Stemme et al.,1995, *supra).* The degree of oxidation was evaluated by a spectro-fluorometric analysis (excitation at 350nm and emission at 433nm) as described by Steinbrecher (Steinbrecher UP. Oxidation of human low density lipoprotein results in derivatization of lysine residues of apolipoprotein B by lipid peroxide decomposition products. J Biol Chem 1987;262:3603-3608). The fluorescent intensity of the oxLDL preparations was always >0.5 as compared to the fluorescent intensity of native LDL <0.2.
Peptides of native and copper-oxidized LDL were generated by proteolysis of lipoproteins (2mg) with agarose-bound α-chymotrypsin (20 µg enzyme; Sigma) in 150 mM NH₄HCO₃ for 6 days at 37°C. The α-chymotrypsin was removed by centrifugation for 10 min at 5000 g, and the apoB100 peptides were isolated from the lipoproteins by ultracentrifugation (Amicon-50) and lyophilised.

### Animals

Seven week-old male apoB-TG mice (B6,129-Apob^{tm2S}gy, DNX Transgenics, Princepton, NJ, USA) were immunized with 50 µg human oxLDL/mouse mixed with equal amount with complete Freund's adjuvant. After 2 weeks, mice were boosted with 50 µg oxLDL mixed with incomplete Freund's adjuvant (v/v). Naive mice were used as donor of spleens for APC preparations.
Five month-old male apoE-KO mice (Jackson Laboratory, Bar Harbor, ME, USA) were fed during 4 months with a pro-atherogenic diet containing 0.15% cholesterol (AB AnalyCen, Linköping, Sweden). Naive mice were used as donors of spleens for antigen presenting cells (APC) preparations and donors of thymus for the preparation of thymocyte-conditioned medium.
Spleens from BALB/c mice (Jackson Laboratory) were used as a source of control I-A^{d} allogenic APCs. For the parallell study, normal B6 mice were immunized with MDA-MSA. A primary immunisation with MDA-MSA in Freund's complete adjuvant was followed by booster injections of MDA-MSA in incomplete adjuvant (see above).

### Cell culture

At sacrifice, the inguinal, mesenteric, aortic and axillary lymph nodes (LN) were harvested. LN cells were prepared by meshing the tissues on 100 µm nylon filters followed by a series of washing steps in Dulbecco's modified Eagle's medium with 5% fetal bovine serum (DMEM/5%). T cell reactivity was determined in 96-well plate assays with 300,000 cells/well in the presence of different stimuli: Concanavalin A (Sigma), native, and oxLDL. Each type of stimulation was performed in triplicate. The plates were incubated for 4 days at 37°C, 5% CO₂. During the last 18 h of culture, cells were pulsed with 1 µCi of ³H-thymidine. Thymidine incorporation was measured with a Wallac Microbeta β-counter (Turku, Finland).
Syngeneic spleen cells used as APCs were prepared as LN cells except that red blood cells in the spleen cell preparations were lysed with ACK lysing buffer (0.15 M NH₄Cl, 1 mM KHCO₃, 0.1 mM Na₂EDTA, pH 7.3).

### T cell hybridomas

T cell hybridomas were generated by polyethylene glycol-induced fusion of LN cells (50x10⁶ cells) with the BW5147 thymoma (30x10⁶ cells) as previously described (Kappler JW, Skidmore B, White J, Marrack P. Antigen-inducible, H-2-restricted, interleukin-2-producing T cell hybridomas. Lack of independent antigen and H-2 recognition. J. Exp. Med. 1981;153:1198-1214). LN cells from oxLDL-immunized apoB-TG mice were stimulated with 3 µg/ml oxLDL during 3 days, while LN cells from apoE-KO mice were not stimulated *in vitro.* After fusion, 1x10⁶ thymocytes/ml were added as feeder cells and the cell suspension was plated in five 96-well plate and incubated at 37°C, 7.5% CO₂. After one day of incubation, hypoxanthine-aminopterin-thymidine was added to the medium to select fused cells. CD3+/CD4+ hybridomas (50,000 cells) were then challenged with concanavalin A, oxLDL, nLDL or MDA-LDL at the indicated doses in presence of 50,000 irradiated spleen cells (matched I-A^{b} haplotype and mismatched I-A^{d} as control). After 24h, 50 µl of supernatant was collected and its IL-2 content was assessed by a CTLL-2 assay. 5,000 CTLL-2 cells were added to the 50 µl of supernatant or to a standard dilution of mouse recombinant IL-2. One µCi of ³H-thymidine was added after one day of incubation and the thymidine was measured with a Wallac Microbeta β-counter after 18 h of culture. Limiting dilution cloning was performed in DMEM/20% supplemented with 5% thymocyte-conditioned medium. Thymocyte-conditioned medium was obtained from the supernatant of 5-day thymocytes culture in DMEM/20%/10mM HEPES/1mM pyruvate. MDA-MSA reactive T cell hybridomas were established in a similar manner.

### RNA and cDNA preparations

mRNA was prepared from 2x10⁶ cells with a Dynabeads mRNA direct kit from Dynal (Oslo, Norway). Single-stranded cDNA synthesis was performed by SuperscriptII (Life Technologies, Täby, Sweden) according to the manufacturer. Briefly, half of the cDNA preparation was incubated with 50 pmol random pdN6 (Pharmacia, Uppsala, Sweden) at 70° for 3 min. After transfer to ice, 5X first strand buffer, SuperscriptII, RNasin (Promega, Madison, WI, USA) and dNTPs (Pharmacia) were added. The samples were incubated for 10 min at 25°C, 50 min at 42°C, 4 min at 94°C and stored at -85°C.

### PCR reactions

cDNA was amplified by PCR in a mastermix containing 10 mM Tris-HCl, 50 mM Kcl, 1.5 mM MgCl₂, 1 mM dNTP, 0.2 U/ml Taq polymerase (Life Technologies, Täby, Sweden) (Pannetier C, Cochet M, Darche S, Casrouge A, Zoller M, Kourilsky P. The sizes of the CDR3 hypervariable regions of the murine T cell receptor beta chains vary as a function of the recombined germ-line segments. Proc. Natl. Acad. Sci. USA. 1993;90:4319-4323). For amplification of 19 TcRα chain cDNA, a set of 19 Vα primers was used together with one Cα primer (Table 1 below). Similarly, 24 VB primers were used with one Cβ primer to amplify the TcR VB chain cDNA (Table 1 below). Final concentration of primers 0.2 µmol/L each. All primer sequence for Vα, Vβ, Cα, and Cβ used in the RT-PCR reactions comes from ref. [Patten, 1984 #344; Chien, 1984 #1273; Chien, 1996 #81; Chou, 1986 #1276; Chou, 1987 #341; Chou, 1987 #1277; Tillinghast, 1986 #1278; Kappler, 1987 #754; Koide, 1993 #338; Pannetier, 1993 #339; Casanova, 1991 #1279]. Each reaction was performed in a separate tube, i.e. each PCR tube contained one of the alternative V primers together with the C primer. The first PCR cycle started with 2.5 min in a hot block at 95°C followed by 56°C (40 sec) and 72°C (60 sec). The ensuing 34 cycles consisted of 94°C (40 sec), 56°C (40 sec) and 72°C (60 sec) in a Tetrad (MJ Research, Wateretown, MA, USA). PCR products were loaded together with a size marker (100 bp, Life Technologies) on a 1.7% agarose gel.

**Table 1: List of primers**

| **Segment** | **PCR primer 5' → 3'** | **Segment** | **Sequencing primer 5' → 3'** |
|---|---|---|---|
| *Vβ1* | CAGTTGATTVGAAATGAGACGGT | *Vβ6* | CTCTCACTGTGACATCTGCC |
| *Vβ2* | AGTCCTGGGGACAAAGAGGTCAAA | | |
| *Vβ3* | AACGATTCTCTGCTGAGTGTCCT | *Cβ* | TGCAATCTCTGCTTTTGATGGCTC |
| *Vβ4* | TTATGGACAATCAGACTGCCTC | | |
| *Vβ5* | GGAGAGAGATAAAGGAAACC | | |
| *Vβ5.2* | AAGGTGGAGAGAGACAAAGGATTC | | |
| *Vβ6* | CTCTCACTGTGACATCTGCC | | |
| *Vβ7* | TAGTAACAGCGAAGGAGACATCCC | | |
| *Vβ8* | CAGGAGGAAACGTGACATTGA | | |
| *Vβ8.1* | CATTACTCATATGTCGCTGAC | | |
| *Vβ8.2* | CATTATTCATATGGTGCTGGC | | |
| *Vβ8.3* | TGCTGGCAACTTCGAATAGGA | | |
| *Vβ9* | TCTCTCTACATTGGCTCTGCA | | |
| *Vβ10* | GCTTCTCACCTCAGTCTTCAGAT | | |
| *Vβ11* | TCCTATAGATGATTCAGGGATGCC | | |
| *Vβ12* | TTATGGAAGATGGTGGGGAT | | |
| *Vβ13* | TGCCCTCGGATCGATTTTCTGCT | | |
| *Vβ14* | ATTACTGTTGGCCAGGTAGAGT | | |
| *Vβ15* | GGCATTTGAACTGATAGCAC | | |
| *Vβ16* | CACTCTGAAAATCCAACCCAC | | |
| *Vβ17* | AGTGTTCCTCGAACTCACAG | | |
| *Vβ18* | AGCCGGCCAAACCTAACATTC | | |
| *Vβ19* | CCCATAAACGGACATAGTTACG | | |
| *Vβ20* | TCTGCAGCCTGGGAATCAGAA | | |
| | | | |
| *Cβ* | TGCAATCTCTGCTTTTGATGGCTC | | |
| *Vα1* | GCACTGATGTCCATCTTCTC | | |
| *Vα2* | AAAGGGAGAAAAAGCTCTCC | | |
| *Vα3* | AAGTACTATTCCGGAGACCC | | |
| *Vα4* | CAGTATCCCGGAGAAGGTC | | |
| *Vα5* | CAAGAAAGACAAACGACTCTC | | |
| *Vα6* | ATGGCTTTCCTGGCTATTGCC | | |
| *Vα7* | TCTGTAGTCTTCCAGAAATC | | |
| *Vα8* | CAACAAGAGGACCGAGCACC | | |
| *Vα9* | TAGTGACTGTGGTGGATGTC | | |
| *Vα10* | AACGTCGCAGCTCTTTGCAC | | |
| *Vα11* | CCCTGCACATCAGGGATGCC | | |
| *Vα12* | TCTGTTTATCTCTGCTGACC | | |
| *Vα13.1* | ACCTGGAGAGAATCCTAAGC | | |
| *Vα34S* | TCCTGGTTGACCAAAAAGAC | | |
| *VαA10* | TGGTTTGAAGGACAGTGGGC | | |
| *VαBWB* | CATTCGCTCAAATGTGAACAG | | |
| *VαBMA* | CAAATGAGAGAGAGAAGCGC | | |
| *VαBMB* | GGAAAATGCAACAGTGGGTC | | |
| *Vα5T* | GACATGACTGGCTTCCTGAAGGCC | | |
| | | | |
| *Cα* | CACCAGCAGGTTCTGGGTTC | | |

Nomenclature and sequences were derived from (Pannetier C, Cochet M, Darche S, Casrouge A, Zoller M, Kourilsky P. The sizes of the CDR3 hypervariable regions of the murine T cell receptor beta chains vary as a function of the recombined germ-line segments. Proc. Natl. Acad. Sci. USA. 1993;90:4319-4323; Patten P, Yokota T, Rothbard J, Chien Y, Arai K, Davis MM. Structure, expression and divergence of T cell receptor beta-chain variable regions. Nature 1984;312:40-46; Chien KR. Genes and physiology: molecular physiology in genetically engineered animals. J Clin Invest 1996;98:S19-S26; Chou HS, Anderson SJ, Louie MC, Godambe SA, Pozzi MR, Behlke MA, Huppi K, Loh DY. Tandem linkage and unusual RNA splicing of the T cell receptor beta-chain variable-region genes. Proc. Natl. Acad. Sci. USA. 1987;84:1992-1996; Kappler JW, Roehm N, Marrack P. T cell tolerance by clonal elimination in the thymus. Cell 1987;49:273-280; and Koide Y, Kaidoh T, Yanagawa T, Yoshida TO. A comparative study on T cell receptor V beta gene usages: spleen cells from the non-obese diabetic (NOD) mouse and its non-diabetic sister strain, the ILI mouse, and infiltrating T cells into pancreata of NOD mice. Microbiol. Immunol. 1993;37:653-659). V primer sequences are from the coding strand and C primer sequences are from the noncoding strand.

### Sequencing of the TcR of the hybridoma 96-6

CDNA was amplified using primers specific for the Vα10 (AACGTCGCAGCTCTTTGCAC) and Vβ6 (CTCTCACTGTGACATCTGCC) chains and with their corresponding C-region primer (Cα: CACCAGCAGGTTCTGGGTTC; CB: TGCAATCTCTGCTTTTGATGGCTC). PCR products were run through a Microcon concentrator (Amicon, Beverly, MA, USA ; membrane cut-off 50,000 Dalton) to separate them from excess of primers and nucleotides before sequencing reaction was performed. The sequencing reaction was performed with the ABI Prism^{™} Dye Terminator Cycle Sequencing Core Kit according to manufacturer (ABI-Perkin Elmer, Sundbyberg, Sweden) using the sequencing primers described previously (Paulsson G, Zhou, X., Törnquist, E., Hansson, G. K. Oligoclonal T cell expansions in atherosclerotic lesions of apoE-deficient mice. Arteriosclerosis, Thrombosis, and Vascular Biology 1999; in press). The amplification started with one cycle at 96°C for 2 min, 55°C for 15 s and 60°C for 4 min, followed by 25 cycles at 96°C for 30 s, 50°C for 15 s and 60°C for 4 min. DNA sequencing products were purified on a Centrisep column (Princeton Separations, Adelphia, NJ, USA). The analysis of the sequencing products was performed with ABI/Perkin Elmer equipment at a core facility at the Center for Molecular Medicine of Karolinska Hospital, Sweden. The sequences were analyzed with the Lasergene program for Macintosh (DNASTAR).

### Modeling of the TcR

The 3D structure was extrapolated from a crystallized mouse TcR (Garcia et al., 1996, *supra).* The α and β chains of this TcR present respectively 79.2% and 73.7% similarity with the α- and β-chain of the 96-7 clone. This score is sufficient to allow an extrapolation of the 3D protein structure of the TcR from the clone 96-7. SwissPdbViewer 3.5 was used for the modelisation and molecular surface images were generated by the Graphical Representation and Analysis of Structure Server (http://trantor.bioc.columbia.edu).

### FACS analysis

Cells were stained for 30 min at 4°C using FITC-conjugated anti-CD3, Cychrome-conjugated anti-CD4, and phycoerythrin-anti-CD8, purified hamster anti-γ/δ and anti-α/β TcR, biotinylated anti-Vβ1, Vβ2, Vβ3.2, Vβ4, Vβ5.1-5.2, Vβ6, Vβ7, VB8.1-8.2, Vβ8.3, Vβ9, Vβ10b, Vβ11, Vβ12, Vβ13 (Phanningen, San Diego, CA, USA). Cells were washed and analyzed with a FACSCalibur® (Becton Dickinson, Mountain View, CA, USA) flow cytometer.

### RESULTS

### T cell hybridomas of oxLDL-immunized apoB-TG mice exhibit I-A^{b} restricted recognition of oxidized LDL

ApoB-TG mice were used to characterize the T cell response to oxLDL. Since these mice constitutively express high levels of human apoB100 from fetal life, they should be tolerant to the native protein. After a primary immunization with human oxLDL in complete and a booster immunization in incomplete Freund's adjuvant, LN cells from apoB-TG mice were reactive specifically against oxLDL and did not respond to native LDL (Fig. 1). T cells were fused with BW5147 lymphoma cells to generate hybridomas. 268 hybridoma cultures were growing, among which 117 were CD3+CD4+. Among these, ten were producing IL-2/IL-4 in response to oxLDL in the presence of syngenic APCs and were cloned by limiting dilution.

Fig. 2 shows representative data from three responding and three non-responding T cell hybridoma clones. The hybridomas as well as the original bulk cultures exhibited I-A^{b} restricted, specific responses to oxLDL but not native LDL. While bulk cultures reacted by IL-2/IL-4 secretion over a relatively wide concentration range of antigen, the T cell hybridomas showed a very narrow concentration range for optimal activation (Fig. 2). It is possible that the wider concentration range of the bulk cultures were caused by additive effects of a large number of T cells with slightly different sensitivities.

### Expression of TcR-Vα10-Jα12/Vβ6-Dβ1.1-Jβ2.7 antigen receptor by oxLDL-reactive T cell hybridomas

The TcR from 96-6 and 96-7, two hybridomas reactive to oxLDL (Fig. 2), were characterized in detail. First, it was determined by FACS analysis that both hybridomas were expressing α/β rather than γ/δ TcR (Fig. 3). The expression of the VB chain of the TcR was then assessed by RT-PCR. Both hybridomas were expressing the Vβ5.2 and Vβ6 variable regions (Fig. 3). The thymoma BW5147 expressed Vβ5.2 rearranged chains and all hybridomas were also expressing Vβ5.2 at the RNA level (data not shown). To certify that no other VB segment than Vβ6 was expressed and to check whether the Vβ5.2 chain was expressed at the surface of the 96-7 clone, a FACS analysis was performed with a panel of anti-VB antibodies. Results obtained with the anti-Vβ5.1-5.2, anti-Vβ11 (as a negative control), and anti-VB6 antibodies are shown Fig. 3. As expected from the data obtained by RT-PCR, the hybridoma was expressing the Vβ6 chain but not the Vβ5.2 chain at its surface.
The complementarity determining region 3 (CDR3), which is the major determinant of epitope binding to the TcR [Davis, 1988 #1280], was sequenced in hybridomas 96-7. Fig. 4 shows the nucleotide sequences of the CDR3 region of the β-chain (which was completely identical to the β-chain sequence of the 96-6) and of the α-chain. The CDR3 region was composed by an association of Vα10-Jα12 and Vβ6-Dβ1.1-Jβ2.7. Thus, CD4⁺ T cells expressing a Vα10-Jα12/Vβ6-Dβ1.1-Jβ2.7-TcR recognize an epitope induced by oxidation of LDL in an I-A^{b} restricted manner. Extrapolation of the 3D structure from a crystallized mouse TcR (clone 2c) (Garcia et al., 1996, *supra)* was possible given the sequence similarity between the α- and β-chains of the 96-7 TcR and the clone 2c TcR (respectively 79.2% and 73.7%; ref. [Rost, 1999 #1281]). Fig. 5 shows the 3D structure of the clone 96-7 TcR and its CDR3 region. This region is composed mainly by hydophobic and polar amino acids.

### The TcR-α10/β6 expressing T cell hybridoma 96-7 recognizes MDA-modified apoB100 protein

The reactivity of hybridoma 96-7 was determined in detail. Since it reacted with oxLDL but not nLDL and the response was I-A^{b} restricted, it was likely to recognize a protein component modified by oxidation. The nature of this modification was unknown but could be caused by fragmentation or adduct formation. 96-7 was therefore exposed to proteolytic peptides of LDL, purified apoB100 or MDA-conjugated apoB100. As shown in Fig. 6, MDA-apoB at 0.1 µg/ml was a strong activator of 96-7 and induced IL-2/IL-4 secretion of the same magnitude as that caused by oxLDL or ConA mitogen. In contrast, apoB100 peptides from native human LDL did not activate 96-7 whereas those from oxidized LDL did. This shows that MDA adduct formation but not fragmentation of apoB100 generated the T cell epitope recognized by the α10/β6-carrying hybridoma 96-7 in the context of I-A^{b}.

### T cell hybridomas from MDA-MSA immunized normal B6 mice specifically recognize malondialdehyde adducts in a model protein

To firmly establish the concept of TcR recognition of aldehyde adducts normal B6 mice were immunized with a model substance, MDA-MSA, and T cell hybridomas were generated. This enabled direct study of TcR recognition of the antigenic epitope. Two different experiments confirmed that the aldehyde adduct itself formed part of the TcR epitope of the hybridoma M20. In the first experiment, M20 cells were coincubated with B6 spleen cells which had been surface-modified with MDA. MDA-modified spleen cells, but not non-modified spleen cells induced IL-2 production by the M20 cells (Fig 8A). In a second experiment, the stimulating capacity of MDA-MSA-pulsed and fixed spleen cells could be blocked by the addition of antibody to MDA but not to control antibody (Fig 8B). Together this shows that (i) the MDA-adducts on peptides can be recognized without processing and (ii) the aldehyde is pointing out from the MHC-bound peptide towards the TcR. This rules out processing-dependent recognition of native self peptides and thus provides direct evidence that aldehyde-adducts can form part of a TcR epitope.

### Naive apoE-KO mice display T cell clones that recognize modified LDL

Having established that immunization of human LDL-tolerant apoB-TG mice with oxLDL activates α10/β6+ CD4+ T cells that specifically recognize MDA-apoB of oxLDL but not native LDL, the question was posed whether similar responses occur by natural immunization in the course of atherosclerosis. ApoE-KO mice were chosen because the aortic atherosclerotic lesions of these mice contain T cells (Zhou X, Stemme S, Hansson GK. Evidence for a local immune response in atherosclerosis. CD4+ T cells infiltrate lesions of apolipoprotein-E-deficient mice. Am. J. Path. 1996;149:359-366).
After fusion between LN cells and BW5147, 220 hybridoma cultures grew out, among which 142 were CD3+/CD4+. These cultures were screened for their reactivity towards human oxLDL, MDA-LDL, and native LDL. Totally 20 cultures were reactive to at least one of the stimuli. Fig. 7 shows the reactivities of five representative cultures. Among them, culture 3a1 exhibited a pattern of reactivity resembling that of the apoB-TG derived hybridoma 96-7, i.e. it responded to MDA-modified LDL (Fig. 7). The fact that both human and mouse MDA-LDL were recognized suggests that the T cell epitope for this hybridoma could be an MDA-modified peptide sequence that is shared between apoB100 of the two species. T cells in culture 3a1 were expressing solely TcRVβ1. This implies that in addition to VB6, other Vβ segments can also be used for recognition of MDA-modified LDL. Culture 6c6, which expressed TcR with the VB segments 4, 8.2, 10 and 13, recognized oxLDL but not MDA-LDL (Fig. 7). This suggests that recognized epitopes consisted oxidation-induced modifications different from MDA adduct formation. Another culture, 6a5, reacted towards nominally native but not oxidized or MDA-modified LDL. It is possible that this culture contained autoreactive T cells recognizing an epitope that is destroyed by oxidation. Alternatively, the epitope might be induced by "minimal modification" and destroyed by further oxidation. Such dynamic changes in epitope formation may also explain the reactivity of culture 4c6, which recognized native and MDA-modified but not oxidized LDL (Fig. 7). In all cases, the reactivities found against human LDL matched those found against mouse LDL suggesting that these T cells were not recognizing heterologous epitopes.

### Discussion

OxLDL accumulates in atherosclerotic plaques (Ylä-Herttuala S, Palinski W, Rosenfeld ME, Parthasarathy S, Carew TE, Butler S, Witztum JL, Steinberg D. Evidence for the presence of oxidatively modified low density lipoprotein in atherosclerotic lesions of rabbit and man. J Clin Invest 1989;84:1086-1095), is taken up by macrophages, and elicits local autoimmune responses in the plaque (Stemme et al., 1995, *supra)* as well as systemically (Palinski et al., 1989, *supra;* and Ylä-Herttula et al., 1989, *supra).* The findings that clonal T cells are activated by oxLDL (Stemme et al., 1995, *supra)* and that T cell-dependent antibody responses to oxLDL develop in atherosclerosis (Zhou X, Paulsson G, Stemme S, Hansson GK. Hypercholesterolemia is associated with a T helper (Th) 1/Th2 switch of the autoimmune response in atherosclerotic apo E-knockout mice. J. Clin. Invest. 1998;101:1717-1725) suggest an important role for cellular immunity in the disease-associated autoimmune response. However, the precise mechanisms governing this response have been unclear. In particular, it has been unclear whether the T cell response to oxLDL' is a "conventional" reaction of TcRαβ towards a peptide presented by MHC class II molecules or a response of T cells expressing either αβ or γ/δ receptors and recognizing lipids presented in the context of CD1 molecules. The latter process has recently been identified as a major mechanism for recognition of microbially derived lipids (Grant EP, Degano M, Rosat JP, Stenger S, Modlin RL, Wilson IA, Porcelli SA, Brenner MB. Molecular recognition of lipid antigens by T cell receptors. J Exp Med 1999;189:195-205).
According to the present invention, using T cell hybridoma technology, it is now demonstrated (i) that mice tolerant to native human LDL develop specific T cell responses to LDL that has undergone oxidation in vitro, (ii) that the responding T cells express the TcRαβ receptor, (iii) may preferentially use TcR carrying the α10 and β6 chains, (iv) are restricted by the MHC class II antigen, I-A^{b}, and (v) recognize MDA-modified apoB100. Finally, it is shown (vi) that similar T cells recognizing oxLDL develop spontaneously in atherosclerotic apoE-KO mice and (vii) that this autoimmunization results in TcR exhibiting several different Vβ domains, suggesting responses to several different, oxidation-induced T cell epitopes.
The use of T cell hybridomas permitted a detailed characterization of the receptors recognizing the antigen and restricting the response. However, they are not useful for assessing the frequency of immune responses in the starting material. Therefore, it is important to correlate the present findings to studies of T cell specificities, phenotypes, and secretory products which are based on whole tissues and primary cultures.

The present analyses confirmed previous results from human T cell clones, which have shown that the T cells recognizing oxLDL are CD4+ TcRαβ+ cells that recognize antigen in the context of MHC class II (Stemme et al., 1995, *supra).* With the use of hybridomas, an actual MHC class II restriction could be demonstrated by showing that T cells of the I-A^{b} haplotype recognized oxLDL presented by I-A^{b} expressing but not I-A^{d} expressing antigen-presenting cells. Notably, the CD1 molecules responsible for presentation of lipid antigens are nonpolymorphic and do not differ between the I-A^{b} and I-A^{d} strains (Balk SP, Bleicher PA, Terhorst C. Isolation and expression of cDNA encoding the murine homologues of CD 1. J Immunol 1991;146:768-774; and Tangri S, Holcombe HR, Casta-no AR, Miller JE, Teitell M, Huse WE, Peterson PA, Kronenberg M. Antigen-presenting function of the mouse CD1 molecule. Ann N Y Acad Sci 1996;778:288-296). The lack of response of the I-A^{b} expressing T hybridoma when exposed to oxLDL presented by I-A^{d} APC therefore argues against CD1 restricted antigen presentation and therefore against reactivity towards the lipid moiety of oxLDL.
I-A molecules present oligopeptides to antigen-specific T cells, which recognize the peptide-I-A complex using TcRαβ. It suggests that the T cell epitope on oxLDL is a modified oligopeptide. This conclusion was confirmed by the finding that hybridoma 96-7 recognized MDA-conjugated apoB100 in the absence of lipid. MDA is formed when unsaturated fatty acid residues are cleaved during lipid peroxidation; it is highly reactive and therefore rapidly interacts with free amino groups in adjacent proteins (Steinbrecher et al., 1987, *supra).* Several other aldehydes exhibit similar activities (Steinbrecher et al., 1987, *supra*) and it is likely that aldehyde adduct formation changes the conformation of apoB100-derived oligopeptides sufficiently to activate cellular immune responses.

The precise nature of the molecular interaction between MDA-apoB and the TcR of T cell hybridoma 96-7 cannot be determined at this stage. Since TcR recognition of lipid peroxidation derived aldehyde adducts has previously not been described it was important to substantiate this concept in an independent system. The present inventors therefore established T cell hybridomas from normal B6 mice immunized with a model substance, MDA-MSA. This enabled direct study of TcR recognition of the antigenic epitope and two different experiments confirmed that the aldehyde adduct itself formed part of the TcR. Furthermore, it is noteworthy that the CDR3 domain is dominated by polar and charged amino acids. This structure would be expected to fit with an oligopeptide exposing charged residues due to MDA crosslinking or possibly with oligopeptide side chains carrying MDA adducts. There is a definite topology of the CDR3 in which the Vα governs the N-terminal region and Vβ seems more responsible for the C-terminal portion of the peptide (Davis MM, Bjorkman PJ. T cell antigen receptor genes and T cell recognition. Nature 1988;334:395-402). These data should facilitate future design of peptides from apoB100 that could be used in vaccination experiments. It will also be important to analyze CDR3 domains of T cells reactive with other immunogenic, oxidatively induced adducts such as 4-hydroxynonenal, to determine whether the CDR3 of aldehyde-peptides carry common motifs used in epitope recognition.

Several factors favor the development of immune responses to oxidized proteins and particles. Firstly, these antigens are taken up by specific scavenger receptors, leading to high intracellular concentration in antigen-presenting cells (Nicoletti A, Caligiuri G, Tornberg I, Kodama T, Stemme S, Hansson GK. The macrophage scavenger receptor type A directs modified proteins to antigen presentation. Eur J Immunol 1999:In press; Abraham R, Singh N, Mukhopadhyay A, Basu SK, Bal V, Rath S. Modulation of immunogenicity and antigenicity of proteins by maleylation to target scavenger receptors on macrophages. J Immunol 1995;154:1-8; and Abraham R, Choudhury A, Basu SK, Bal V, Rath S. Disruption of T cell tolerance by directing a self antigen to macrophage-specific scavenger receptors. J Immunol 1997;158:4029-4035). Macrophages rely on scavenger receptor SR-A to present oxLDL to specific T cells but dendritic cells may use additional scavenger receptors for the same purpose (Nicoletti et al., **1999**, *supra).* This might result in the presentation of different oxidized particles by different antigen-presenting cells, with obvious consequences for the ensuing immune responses.

Secondly, the reactive aldehydes formed during lipid peroxidation may not only participate in the formation of T cell epitopes but also act as costimulants for T cell activation (Rhodes J, Chen H, Hall SR, Beesley JE, Jenlcins DC, Collins P, Zheng B. Therapeutic potentiation of the immune system by costimulatory Schiff-base-forming drugs. Nature 1995;377:71-75). For instance, the Schiff base-forming aldehyde p-hydroxyphenylacetaldehyde, which can be formed by myeloperoxidase attack on lipoproteins (Daugherty A, Dunn JL, Rateri DL, Heinecke JW. Myeloperoxidase, a catalyst for lipoprotein oxidation, is expressed in human atherosclerotic lesions. J Clin Invest 1994;94:437-444), provides a costimulatory signal to CD4+ T cells by activating K⁺ channels (Rhodes et al., 1995, *supra).* This type of activation appears to be biased towards Thl effector responses (Rhodes et al., **1995,** *supra),* which predominate in atherosclerotic lesions (Zhou et al., **1998,** *supra*; and Geng YJ, Holm J, Nygren S, Bruzelius M, Stemme S, Hansson GK. Expression of the macrophage scavenger receptor in atheroma. Relationship to immune activation and the T-cell cytokine interferon-gamma. Arterioscler. Thromb. 1995;15:1995-2002). Thus, lipoprotein oxidation in the arterial intima promotes proinflammatory autoimmunity both by generating antigen, inducing ligands for uptake by antigen-presenting cells, and forming costimulatory factors that promote T cell activation to proinflammatory Thl effector cells. The current experimental design using T cell hybridomas is not informative with regard to effector functions since the original cytokine secretory pattern may not be preserved after fusion. Therefore, further studies using primary cultures and in situ staining will be important to elucidate these aspects of immune activity in atherosclerosis.

## Claims

1. A method of producing a vaccine against atherosclerosis, which comprises the steps of
(a) providing a library of peptides derived from the protein apoB100;
(b) incubating said peptides with aldehydes to produce conjugates of peptide-aldehyde;
(c) separating non-conjugated peptides and aldehydes from the library;
(d) incubating the conjugates obtained from step (c) with a panel of MHC class II proteins;
(e) selecting conjugates capable of binding to the MHC class II proteins corresponding to the MHC haplotype of the individual to be vaccinated;
(f) formulating a vaccine composition of conjugates selected according to step (e) and a pharmaceutically acceptabler carrier.

2. Use of a T cell receptor (TcR) comprising an amino acid sequence as defined by SEQ ID NO:4 and SEQ ID NO:2, or a functional analogue thereof, as a lead compound in a method of molecular modelling of a ligand capable of binding said TcR, said three dimensional structure being **characterised by** a flattened surface and the presence of exposed surface charges.

3. A method for diagnosis of affliction with atherosclerosis in a patient, by which method the presence of T cells that recognize oxidized LDL is detected, said method comprising the steps of:
(a) combining a sample of body fluid obtained from said patient with a composition, comprised of a peptide, which is derived from apoB100, conjugated with an aldehyde, and capable of eliciting an immune response against non-native oxidized low density lipoprotein (LDL) in a subject by interacting with TcR;
(b) detecting the relative presence in said sample of T cells that are activated by exposure to said aldehyde-peptide conjugate;
(c) comparing the detected presence of reacting T cells to a reference value; and
(d) using the comparison in (c) in the diagnosis of affliction with atherosclerosis.

4. A method according to claim 27, wherein in step (c), the proportion of the total amount of T cells which constitutes TcR comprising an amino acid sequence as defined by SEQ ID NO:4 and SEQ ID NO:2, is determined.

5. A method according to claim 27 or 28, wherein said body fluid sample is a blood or cell sample.

6. A method according to any one of claims 27-29, wherein said marker is an antibody, preferably a monoclonal antibody.

## Patentansprüche

1. Verfahren zum Produzieren eines Impfstoffs gegen Atherosklerose, das die folgenden Schritte umfasst:
(a) Bereitstellen einer Bibliothek von Peptiden, die vom Protein apoB100 abgeleitet sind;
(b) Inkubieren der Peptide mit Aldehyden, um Konjugate von Peptid-Aldehyd zu produzieren;
(c) Trennen von nicht konjugierten Peptiden und Aldehyden von der Bibliothek;
(d) Inkubieren der Konjugate, die aus Schritt c) erhielten wurden, mit einer Auswahl von MHC-Klasse-II-Proteinen;
(e) Auswählen von Konjugaten, die an die MHC-Klasse-II-Proteine binden können, die dem MHC-Haplotyp der Einzelperson entsprechen, die geimpft werden soll;
(f) Formulieren einer Impfstoffzusammensetzung von Konjugaten, die gemäß Schritt (e) ausgewählt wurden, und einem pharmazeutisch unbedenklichen Trägerstoff.

2. Verwendung eines T-Zell-Rezeptors (T cell receptor, TcR), umfassend eine Aminosäurensequenz, wie von SEQ ID NO: 4 und SEQ ID NO: 2 definiert, oder eines funktionellen Analogons davon als eine Ausgangsverbindung in einem Verfahren zum molekularen Modellieren eines Liganden, der den besagten TcR binden kann, wobei die dreidimensionale Struktur durch eine abgeflachte Oberfläche und das Vorliegen von exponierten Oberflächenladungen **gekennzeichnet** ist.

3. Verfahren zur Diagnose von Beschwerden mit Atherosklerose in einem Patienten, wobei mit diesem Verfahren das Vorliegen von T-Zellen, die oxidiertes LDL erkennen, nachgewiesen wird, wobei das besagte Verfahren die folgenden Schritte umfasst:
(a) Vereinen einer Probe von Körperflüssigkeit, die von dem besagten Patienten bezogen wurde, mit einer Zusammensetzung, die sich aus einem Peptid zusammensetzt, das von apoB100 abgeleitet ist, mit einem Aldehyd konjugiert ist und eine Immunantwort auf nicht natives, oxidiertes Lipoprotein mit geringer Dichte (low density lipoprotein, LDL) in einem Probanden durch Interagieren mit TcR auslösen kann;
(b) Nachweisen des relativen Vorliegens von T-Zellen, die durch Exposition gegenüber dem besagten Aldehyd-Peptid-Konjugat aktiviert werden, in der besagten Probe;
(c) Vergleichen des nachgewiesenen Vorliegens von reagierenden T-Zellen mit einem Bezugswert und
(d) Verwenden des Vergleichs in (c) in der Diagnose von Beschwerden mit Atherosklerose.

4. Verfahren nach Anspruch 3, wobei in Schritt (c) der Anteil der Gesamtmenge von T-Zellen, der TcR, umfassend eine Aminosäurensequenz, wie von SEQ ID NO: 4 und SEQ ID NO: 2 definiert, ausmacht, bestimmt wird.

5. Verfahren nach Anspruch 3 oder 4, wobei die besagte Körperflüssigkeitsprobe eine Blut- oder Zellprobe ist.

6. Verfahren nach einem der Ansprüche 3 - 5, wobei der besagte Marker ein Antikörper, vorzugsweise ein monoklonaler Antikörper ist.

## Revendications

1. Procédé de production d'un vaccin contre l'athérosclérose, qui comprend les étapes suivantes :
(a) fournir une banque de peptides dérivés de la protéine apoB100 ;
(b) incuber lesdits peptides avec des aldéhydes pour produire des conjugués de peptide-aldéhyde ;
(c) séparer les peptides et les aldéhydes non conjugués de la banque ;
(d) incuber les conjugués obtenus à partir de l'étape (c) avec une collection de protéines du CMH de classe II ;
(e) sélectionner les conjugués capables de se lier aux protéines du CMH de classe II correspondant à l'haplotype du CMH de l'individu à vacciner ;
(f) formuler une composition vaccinale des conjugués sélectionnés selon l'étape (e) et d'un support pharmaceutiquement acceptable.

2. Utilisation d'un récepteur des cellules T (TcR) comprenant une séquence d'acides aminés telle que définie par SEQ ID n° 4 et SEQ ID n° 2, ou d'un analogue fonctionnel de celui-ci, comme composé de tête dans un procédé de modelage moléculaire d'un ligand capable de se lier au dit TcR, ladite structure tridimensionnelle étant **caractérisée par** une surface aplatie et la présence de charges de surface exposées.

3. Procédé de diagnostic d'une affection avec athérosclérose chez un patient, procédé par lequel la présence de cellules T qui reconnaissent les LDL oxydées est détectée, ledit procédé comprenant les étapes consistant à :
(a) combiner un échantillon de liquide corporel obtenu dudit patient avec une composition composée d'un peptide qui est dérivé de l'apoB100, conjugué avec un aldéhyde, et capable de déclencher une réponse immunitaire contre des lipoprotéines de basse densité (LDL) oxydées non natives chez un sujet par interaction avec le TcR ;
(b) détecter la présence relative dans ledit échantillon de cellules T qui sont activées par une exposition au dit conjugué aldéhyde-peptide ;
(c) comparer la présence détectée de cellules T réactives avec une valeur de référence ; et
(d) utiliser la comparaison dans (c) dans le diagnostic d'une affection avec athérosclérose.

4. Procédé selon la revendication 3, dans lequel dans l'étape (c), la proportion de la quantité totale de cellules T qui constitue le TcR comprenant une séquence d'acides aminés telle que définie par SEQ ID n° 4 et SEQ ID n° 2, est déterminée.

5. Procédé selon la revendication 3 ou 4, dans lequel ledit échantillon de liquide corporel est un échantillon de sang ou cellulaire.

6. Procédé selon l'une quelconque des revendications 3 à 5, dans lequel ledit marqueur est un anticorps, de préférence un anticorps monoclonal.


### SEQUENCE LISTING

<110> KAROLINSKA INNOVATIONS AB
<120> ATEROSCLEROSIS VACCINE
<130> 57301
<140>
   <141>
<160> 4
<170> PatentIn Ver. 2.1
<210> 1
   <211> 96
   <212> DNA
   <213> Mouse
<220>
   <221> CDS
   <222> (1)..(96)
<400> 1
   ttt ctc tgt gcc agc agt ata caa agc tcc tat gaa cag tac ttc ggt   48
   Phe Leu Cys Ala Ser Ser Ile Gln Ser Ser Tyr Glu Gln Tyr Phe Gly
     1               5                  10                  15
   ccc ggc acc agg ctc acg gtt tta gag gat ctg aga aat gtg act cca   96
   Pro Gly Thr Arg Leu Thr Val Leu Glu Asp Leu Arg Asn Val Thr Pro
                20                  25                  30
<210> 2
   <211> 32
   <212> PRT
   <213> Mouse
<400> 2
   Phe Leu Cys Ala Ser Ser Ile Gln Ser Ser Tyr Glu Gln Tyr Phe Gly
     1               5                  10                  15
   Pro Gly Thr Arg Leu Thr Val Leu Glu Asp Leu Arg Asn Val Thr Pro
                20                  25                  30
<210> 3
   <211> 96
   <212> DNA
   <213> Mouse
<220>
   <221> unsure
   <222> (54)
<220>
   <221> CDS
   <222> (1)..(96)
<400> 3
   tca ggc act tat ctc tgt gct ata gat ccg agg ggc tac aaa ctt acc   48
   Ser Gly Thr Tyr Leu Cys Ala Ile Asp Pro Arg Gly Tyr Lys Leu Thr
    1               5                  10                  15
   ttc ggg aca gga aca agc ttg ttg gtt gat cca aac atc cag aac cca   96
   Phe Gly Thr Gly Thr Ser Leu Leu Val Asp Pro Asn Ile Gln Asn Pro
               20                  25                  30
<210> 4
   <211> 32
   <212> PRT
   <213> Mouse
<400> 4
   Ser Gly Thr Tyr Leu Cys Ala Ile Asp Pro Arg Gly Tyr Lys Leu Thr
    1               5                  10                  15
   Phe Gly Thr Gly Thr Ser Leu Leu Val Asp Pro Asn Ile Gln Asn Pro
               20                  25                  30